# EUROPEAN PATENT APPLICATION

(11) **EP 4 000 507 A1**
(43) Date of publication of application: **25.05.2022**
(21) Application number: 21209976.6
(22) Date of filing: 23.11.2021
(51) Int. Cl.: A61B 5/021, A61B 5/00

(54) **METHOD AND APPARATUS FOR MEASURING PHYSIOLOGICAL PARAMETER**

(30) Priority: 23.11.2020 CN 202011321130
(71) Applicant: Shenzhen Mindray Bio-Medical Electronics Co., Ltd., Shenzhen, Guangdong 518057 (CN)
(72) Inventor: QING, Lei, Shenzhen, 518057 (CN); CAO, Jianfang, Shenzhen, 518057 (CN); JIANG, Xia, Shenzhen, 518057 (CN); HE, Zhilu, Shenzhen, 518057 (CN)
(74) Representative: KIPA AB

(57) **Abstract**

A method for measuring physiological parameter, including: displaying a vital sign monitoring interface, the vital sign monitoring interface comprises at least two measurement start control; according to the triggering operation input by a user through the measurement start control, determining the position information corresponding to the triggered measurement start control; after acquiring a blood pressure value collected by a blood pressure measurement accessory, associating the blood pressure parameter value with the position information and displaying the blood pressure parameter value in the vital sign monitoring interface by indicating the correspondence relationship between the blood pressure parameter value and the position information, to remind the user that the blood pressure parameter value is in which position of the monitored subject. The user only needs to trigger the measurement start control, and the disclosure can automatically associate the collected blood pressure parameter value with the position information, which reduces the user's operation steps and has a better user experience. In addition, this disclosure also provides vital sign monitoring equipment and readable storage media.

## Description

### CROSS REFERENCE

This disclosure claims the priority of Chinese Patent Application No. 202011321130.7, filed on November 23, 2020. The application is hereby incorporated by reference.

### TECHNICAL FIELD

This disclosure relates to the technical field of medical equipment, and more particularly to a method and apparatus for measuring physiological parameter.

### BACKGROUND

For getting more accurately access and record the physiological parameter value, it is necessary to perform multiple measurements with different postures or different part, when the apparatus, which monitoring physiological parameter, performs common test on the physiological parameter of the monitored object. And we can get multiple measurement results of the same physiological parameter. Taking blood pressure measurement as an example, it is usually necessary to measure the blood pressure value of the left arm of the patient in the lying posture and the blood pressure value of the right arm in the lying posture. The two blood pressure measurement results can more comprehensively evaluate the patient's blood pressure status.

The vital sign monitoring equipment usually can only guide the user to measure the physiological parameter in one position, and the measurement results in other positions need to be manually input by medical staff, which is relatively inconvenient to operate.

### SUMMARY OF THIS DISCLOSURE

In view of this, the disclosure provides a vital sign monitoring equipment and a method for measuring physiological parameter to realize the automatic measurement of the physiological parameter of a monitored object in a variety of different positions, reduce a manual operation of medical staff, and improve the convenience of measurement.

In a first aspect, an embodiment provides a method for measuring physiological parameter, including: displaying a vital sign monitoring interface, the vital sign monitoring interface including at least two measurement start control.

In response to a triggering operation input by a user through the target measurement start control, one of at least two measurement start control, a target position information is determined corresponding to the target measurement start control; the target position information is used to indicate a target posture that a monitored object needs to maintain and/or a target measurement position of the monitored object during blood pressure measurement.

A target blood pressure parameter value of the monitored object is obtained by a blood pressure measurement accessory.

A correspondence relationship is established between the target blood pressure parameter value and the target position information.

The target blood pressure parameter value is displayed by indicating the correspondence relationship between the target blood pressure parameter value and the target position information, in the vital signs monitoring interface.

In a second aspect, an embodiment provides a method for measuring physiological parameter, including: displaying a vital sign monitoring interface, which including at least two measurement start control.

In response to a triggering operation input by a user through a target measurement start control, one of at least two measurement start control, determining a target position information corresponding to the target measurement start control; the target position information is used to indicate a target posture that a monitored object needs to maintain and/or the target measurement position of the monitored object during physiological sign parameter measurement.

A target physiological parameter value of the monitored object is obtained by a physiological parameter measurement accessory.

A correspondence relationship is established between the target physiological parameter value and the target position information.

The target physiological parameter value is displayed by indicating the correspondence relationship between the target physiological parameter value and the target position information in the vital signs monitoring interface.

In a third aspect, the embodiments of the present disclosure provide a method for measuring physiological sign parameters, including: displaying a vital sign monitoring interface which including a measurement start control and the measurement start control corresponds to the target physiological parameter.

In response to a triggering operation input by a user through the measurement start control, controlling the physiological parameter measurement accessory to collect parameter data of the target physiological parameter of a monitored object multiple times at different measurement moments.

Parameter data of the target parameter of the monitored object, which collected multiple times, is displayed in the vital sign monitoring interface.

In a fourth aspect, an embodiment of the disclosure provides a vital sign monitoring device, including: a display that is configured to display a vital sign monitoring interface, and the vital sign monitoring interface includes at least two measurement start control; in the vital sign monitoring interface, a target blood pressure parameter value is displayed by indicating a correspondence relationship between the target blood pressure parameter value and a target position information.

A blood pressure measurement accessory that is used to collect the target blood pressure parameter value of a monitored object.

A processor that is configured to: determine the target position information corresponding to the target measurement start control in response to a triggering operation input by a user through a target measurement start control of the at least two measurement start control; where the target position information is used to indicate a target posture that a monitored object needs to maintain and/or a target measurement position of a monitored object when performing blood pressure measurement.

The target blood pressure parameter value of the monitored object, which collected by the blood pressure measurement accessory, is obtained.

A correspondence relationship is established between the target blood pressure parameter value and the target position information.

In a fifth aspect, an embodiment of the disclosure provides a vital sign monitoring equipment, including: a display that is configured to display a vital sign monitoring interface, and the vital sign monitoring interface includes at least two measurement start control; in the vital sign monitoring interface, a target physiological parameter value is displayed by indicating a correspondence relationship between the target physiological parameter value and a target position information.

A physiological parameter measurement accessory that is used to collect the target physiological parameter value of a monitored object.

In response to a user's triggering operation input by a user through a target measurement start control of the at least two measurement start control, a processor determines the target position information corresponding to the target measurement start controls; wherein the target position information is used to indicate a target posture and/or a monitored object's target measurement position of a monitored object when performing physiological parameter measurement.

The target physiological parameter value of the monitored object, which collected by physiological parameter measurement accessory, is obtain.

A correspondence relationship is established between the target physiological parameter value and target position information.

In a sixth aspect, an embodiment of the disclosure provides a vital sign monitoring equipment, including: a display that is configured to display a vital sign monitoring interface, and the vital sign monitoring interface comprises measurement start control which corresponds to a target physiological parameter.

The parameter data of the target physiological parameters of the monitored object, which collected multiple times, is displayed in the vital sign monitoring interface.

A blood pressure measurement accessory that is used to collect the parameter data of the target physiological parameters of the monitored object.

In response to a triggering operation input by a user through the measurement start control, a processor control a physiological parameter measurement accessory to collect the parameter data of the target physiological parameter of the monitored object multiple times at different measurement moments.

In a seventh aspect, an embodiment of the disclosure provides a readable storage medium on which a computer program is stored, and when the computer program is loaded and executed by a processor, the physiological parameter measurement method described in any of the above aspects is implemented.

It can be seen from the above technical solutions that a display vital sign monitoring interface includes at least two measurement start control in physiological parameter monitoring solution, provided by this disclosure.

Based on a user's triggering operation on the measurement start control, the position information is determined corresponding to the triggered measurement start control.

After obtaining a blood pressure parameter value collected by a blood pressure measurement accessory, the blood pressure parameter value is associated with the position information.

When the blood pressure parameter value is displayed on the vital sign monitoring interface, the user is prompted to the blood pressure parameter value of the monitored object and the position corresponding to the blood pressure parameter value by displaying the correspondence relationship between the blood pressure parameter value and the position information.

In some embodiments, it including: storing the target blood pressure parameter value, the target position information, and the correspondence relationship between the target blood pressure parameter value and the target position information.

In some embodiments, said displaying the target blood pressure parameter value by indicating the correspondence relationship between the target blood pressure parameter value and the target position information, including: determining a display area corresponding to the target position information in the vital sign monitoring interface, and displaying the target blood pressure parameter value in the display area corresponding to the target position information.

In some embodiments, the vital sign monitoring interface includes at least two position information icons; the at least two position information icons are used to indicate the position information that needs to be maintained during blood pressure measurement, and different position information icons and different measurement start control have a pre-set correspondence relationship.

In some embodiments, said determining the target position information corresponding to the target measurement start control, includes: obtaining a pre-set correspondence relationship between a measurement start control and position information; according to the pre-set correspondence relationship, the target position information is determined corresponding to the target measurement start control.

In some embodiments, the measurement start control includes common measurement start control, common position information icon, and measurement start control; the common measurement start control is associated with the common position information icon, and the common position information icon is used to indicate pre-set conventional position information that needs to be maintained during blood pressure measurement.

In some embodiments, said determining the target position information corresponding to the target measurement start control, includes: when the target measurement start control is the measurement start increase control, a position information setting interface is displayed; the position information is received that set by the user for the target measurement start control according to the position information setting interface; the set position information is determined as the target position information corresponding to the measurement start increase control.

In some embodiments, said displaying the target blood pressure parameter value by indicating the correspondence relationship between the target blood pressure parameter value and the target position information, includes: when the target measurement start control corresponding to the target position information is the measurement start increase control, a target position information icon is generated for representing the target position information; the target position information icon is displayed, a display area is determined corresponding to the target position information in the vital signs monitoring interface, and the target blood pressure parameter value is displayed in the display area corresponding to the target position information.

In some embodiments, said after establishing correspondence relationship between target blood pressure parameter values and target position information, further includes: determining the target position information associated with a pre-set blood pressure difference calculation rule; calculating, by the pre-set blood pressure difference calculation rule, a blood pressure difference between the target blood pressure parameter values corresponding to associated target position information; displaying the blood pressure difference in the vital sign monitoring interface.

In some embodiments, said displaying the blood pressure difference, includes: if the blood pressure difference meets the blood pressure alarm condition, the blood pressure difference is displayed as a pre-set alarm pattern.

In some embodiments, it further includes: when there are multiple target blood pressure parameter values and each target blood pressure parameter values corresponding to different target position information, displaying, by a pre-set highlight way, a last collected target blood pressure parameter value and the target position information, which is corresponding to the last collected target blood pressure parameter value.

In some embodiments, it further includes: in response to a modification instruction to modify the target position information, a modified position information is obtained; the target position information is replaced with the modified position information.

In some embodiments, it further includes: before establishing a correspondence between the target blood pressure parameter value and the target position information; an actual position information of the monitored object is determined when the target blood pressure parameter value is collected; determining whether the actual position information is consistent with the target position information; wherein establishing a correspondence relationship between the target blood pressure parameter value and the target position information, includes: when the actual position information is consistent with the target position information, establishing the correspondence relationship between the target blood pressure parameter value and the target position information.

In some embodiments, it further includes: when the actual position information is inconsistent with the target position information, outputting a prompt message.

In some embodiments, each measurement start controls corresponds to different blood pressure measurement accessory, and the vital sign monitoring interface includes prompt information for indicating the correspondence relationship between the measurement start control and the blood pressure measurement accessory.

In some embodiments, it further includes: generating and storing a physiological parameter measurement mode, wherein the physiological parameter measurement mode includes at least one of: the vital sign monitoring interface, the correspondence relationship between the target measurement start control and the target position information, and a correspondence relationship between the blood pressure measurement accessory and the target position information; in response to an instruction to invoke the physiological parameter measurement mode, executing the physiological parameter measurement mode.

In an embodiment, a method for measuring physiological parameter, includes: displaying a vital sign monitoring interface which including at least two measurement start control; in response to a triggering operation input by a user through a target measurement start control of the at least two measurement start control, a target position information is determined corresponding to the target measurement start control; the target position information is used to indicate a target posture that a monitored object needs to maintain and/or a target measurement position of a monitored object during blood pressure measurement; a target physiological parameter value of the monitored object is obtained by a physiological parameter measurement accessory; a correspondence relationship is established between the target physiological parameter value and the target position information; the target physiological parameter value is displayed in the vital signs monitoring interface, the target physiological parameter value and the target position information.

In an embodiment, a method for measuring physiological parameter, includes: displaying a vital sign monitoring interface. The vital sign monitoring interface including a measurement start control that corresponds to the target physiological parameter. in response to a triggering operation input by a user through the measurement start control, controlling a physiological parameter measurement accessory to collect parameter data of the target physiological parameter of a monitored object's multiple times at different measurement moments; displaying the parameter data of the target physiological parameters of the monitored object collected multiple times in the vital sign monitoring interface.

In some embodiments, the display is further configured to display the blood pressure difference according to a pre-set alarm pattern when the blood pressure difference meets a blood pressure alarm condition.

In some embodiments, the processor is further configured to obtain modified position information in response to a modification instruction to modify the target position information and then replace the target position information with the modified position information.

In some embodiments, the processor is further configured to output prompt information, when the actual position information is inconsistent with the target position information.

The user only needs to trigger a measurement start control, and the disclosure can automatically associate the collected blood pressure parameter value with the position information, which reduces the user's operation steps and has a better user experience.

### BRIEF DESCRIPTION OF THE DRAWINGS

In order to more clearly illustrate the technical solutions in the embodiments of this disclosure or in the prior art, a brief introduction to the drawings required for the description of the embodiments or the prior art will be provided below. Obviously, the drawings in the following description are only some of the embodiments of this disclosure, and those of ordinary skilled persons in the art would also have been able to obtain other drawings from these drawings without involving any inventive effort.
FIG. 1 is a schematic flow diagram of a method for monitoring physiological sign parameters;
FIG. 2 is a schematic diagrams of a vital sign monitoring interface;
FIG. 3 is a schematic diagrams of a vital sign monitoring interface;
FIG. 4 is a schematic diagrams of a vital sign monitoring interface;
FIG. 5 is a schematic structural diagram of a vital signs monitoring apparatus;
FIG. 6 is a schematic structural diagram of a monitoring apparatus;

### DETAILED DESCRIPTION

The technical solutions of the embodiments of this disclosure will be described below clearly and comprehensively in conjunction with the accompanying drawings of the embodiments of this disclosure. Obviously, the embodiments described are merely some of, rather than all of, the embodiments of this disclosure. Based on the embodiments in this disclosure, all the other embodiments that would have been obtained by those of ordinary skill in the art without any inventive effort shall fall within the scope of protection of this disclosure.

In the medical field, it is necessary to measure a physiological parameter values of a same physiological parameter of the patient in different situations, the current vital sign monitoring apparatus can only obtain a physiological parameter value in one situation in one measurement process, and physiological parameter value in other situation needs to be manually input by medical staff, which is relatively inconvenient to operate. For example, in order to assess and record the blood pressure of a new patient in the general ward of a hospital, the blood pressure value of the patient in a certain position is often measured by the vital sign monitoring equipment, and the blood pressure value of other positions needs to be manually supplemented by the medical staff.

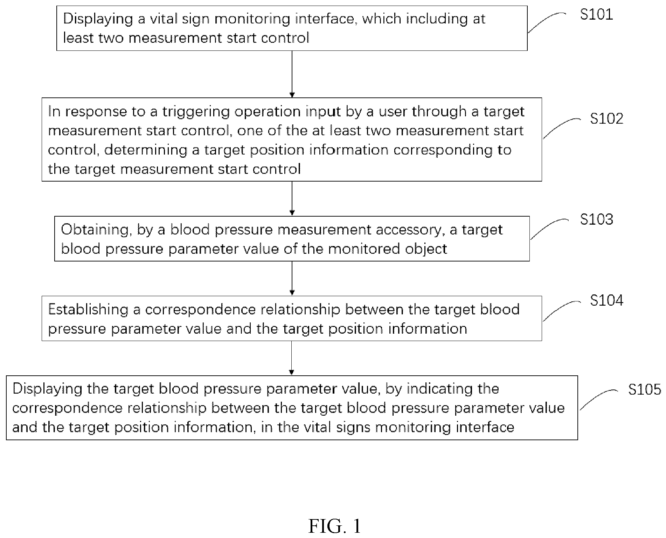

In order to simplify the monitoring operation process of medical staff, the embodiment of the disclosure provides a method for measuring physiological parameter. Taking the measurement of blood pressure parameters as a specific embodiment, the flow of the method for measuring the physiological parameters will be described. As shown in FIG. 1, an embodiment of the method for measuring physiological parameter includes steps S101-S105. It should be noted that the device to which this method embodiment is applied may be referred to as a vital sign monitoring equipment.

At step 101, display a vital sign monitoring interface, and the vital sign monitoring interface includes at least two measurement start controls.

Wherein the vital signs monitoring interface may be a part of a monitoring main interface, or may be a display interface independent of the monitoring main interface, which is associated with the monitoring main interface in a floating, embedded, hidden manner, etc. The vital sign monitoring interface may include a display area of at least one physiological parameter for displaying measurement data of the physiological parameter. For example, the measurement data may include at least one of a parameter value, a parameter waveform, and a parameter trend graph. It should be noted that the vital sign monitoring interface also includes at least two measurement start controls, and the user can instruct the vital sign monitoring equipment to collect blood pressure parameters by triggering the measurement start control.

At step 102, in response to a triggering operation input by a user through a target measurement start control, one of at least two measurement start control, determining the target position information corresponding to the target measurement start control.

Wherein the user can trigger the measurement start control of at least two measurement start controls, and it should be noted that the user can trigger a single measurement start control or multiple measurement start controls at the same time. For ease of description, the measurement start control triggered by the user can be referred to as the target measurement start control. The user can trigger the measurement start control directly through the touch screen, or indirectly trigger the measurement start control through physical buttons such as the buttons set on an equipment body or the buttons set on a blood pressure measurement accessory.

It is understandable that the user triggers the measurement start control to instruct a vital sign monitoring equipment to collect a blood pressure parameter value of the monitored object in a one position. In order to enable the vital sign monitoring equipment to automatically associate the blood pressure parameter value with a position information after collecting the blood pressure parameter value, it is necessary to determine in advance the position of the monitored object when the blood pressure parameter value is collected. Therefore, after the vital signs monitoring equipment receives the triggering operation on the target measurement start control, it needs to determine the position information corresponding to the target measurement start control. For ease of description, the position information may be referred to as target position information.

A target position information is used to indicate the target position that the monitored object needs to maintain when performing blood pressure measurement and/or the target measurement body part of the monitored object. Taking blood pressure measurement as an example, the target position can be lying or standing, and the target measurement body part can be the left hand, right hand, left leg, or right leg.

There are many ways to determine the target position information. For example, in one embodiment, when there is a pre-set correspondence relationship between the measurement start control and the position information, the target position information corresponding to the target measurement start control is determined according to the correspondence relationship; As another example, in another embodiment, the user may be prompted to set the position information corresponding to the target measurement start control, and the position information set by the user may be determined as the target position information; As another example, in another embodiment, there is a pre-set correspondence relationship between the blood pressure measurement accessory and the position information, detecting the measurement state of the blood pressure measurement accessory and view the position information corresponding to the blood pressure measurement accessory in the measurement state as the target position information. For the specific implementation process of the multiple determination methods, please refer to the following description in conjunction with the accompanying drawings. Of course, the target position information can also be determined in other ways that those skilled in the art can think of.

At step 103, obtaining, by a blood pressure measurement accessory, a target blood pressure parameter value of the monitored object; wherein the vital signs monitoring equipment including a blood pressure measurement accessory. In response to a triggering operation input by a user through a target measurement start control, the blood pressure measurement accessory collects the blood pressure parameter value of the monitored object. For ease of description, the collected blood pressure parameter value may be referred to as the target blood pressure parameter value. It should be noted that the blood pressure measurement accessory can perform blood pressure measurement according to any existing blood pressure measurement method, which is not specifically limited in the embodiment of this disclosure.

This step is not limited to being executed after step step 102. After receiving the triggering operation input by the user through the target measurement start control, the target blood pressure parameter value can be collected.

At step 104, establishing the correspondence relationship between the target blood pressure parameter value and the target position information.

It can be understood that the target position information determined in step S102 is a certain position of the monitored object, and in step S103, the blood pressure measurement accessory collects blood pressure parameter values of the monitored object in this position. In order to show the association between the position information and the blood pressure parameter value, the vital sign monitoring equipment associates the position information with the blood pressure parameter value, that is, establishes the correspondence relationship between the target blood pressure parameter value and the target position information.

Step 105, in a vital sign monitoring interface, display a target blood pressure parameter value by indicating a correspondence relationship between a target blood pressure parameter value and a target position.

Wherein, in the vital sign monitoring interface, display the target blood pressure parameter value by indicating the correspondence relationship between the target blood pressure parameter value and the target position information for reminding the user in which position the target blood pressure parameter value was collected.

For example, in one embodiment, a display area corresponding to a target position information is determined in a vital sign monitoring interface, and a target blood pressure parameter value is displayed in the display area corresponding to the target position information. Specifically, the vital sign monitoring interface can display the target position information, and in the display area corresponding to the target position information, the target blood pressure parameter value is displayed in a position corresponding to the display position of the target position information. As another example, a parameter value display style corresponding to a position information can be pre-set, therefore, when a target blood pressure parameter value is displayed, adding the parameter value display style corresponding to the target blood pressure parameter value. Since different position information corresponds to different parameter value display styles, the user can identify the target position information corresponding to the target blood pressure parameter value according to the parameter value display style of the target blood pressure parameter value.

It can be seen from the above technical solutions that, in a physiological parameter measurement method provided in the embodiments of this disclosure, a vital sign monitoring interface includes at least two measurement start controls. The user can trigger any one or more of the measurement start control. Based on the user's triggering operation, this embodiment can determine the position information corresponding to the triggered measurement start control. After obtaining the blood pressure parameter value collected by the blood pressure measurement accessory, associating the blood pressure parameter value with the position information. And when the blood pressure parameter value is displayed on the vital signs monitoring interface, using display mode that can indicate the correspondence relationship between the blood pressure parameter value and the position information, so as to remind the user that the blood pressure parameter value is the blood pressure parameter value in which position of a monitored object. In actual applications, the user only needs to trigger the measurement start control, and the embodiment of this disclosure can automatically associate the collected blood pressure parameter value with the position information, what can reduce the user operation steps. the measurement operation is simpler and more convenient, and the user experience is better.

Further, after obtaining the correspondence relationship between the target blood pressure parameter value and the target position information in step S104 of the previous embodiment, and storing a target blood pressure parameter value, a target position information and the correspondence relationship between the target blood pressure parameter value and the target position information. For example, automatic storage or storage based on a save operation entered by the user. In addition, it can be saved.

In addition, it can be saved locally in the vital signs monitoring equipment, or uploaded to other equipment such as monitoring equipment, central monitoring station, Clinical Information System (CIS) server, Hospital Information System (HIS) server Or Electronic Medical Record (EMR) server, etc. for storage. In the embodiment of the disclosure, not only a measurement operation interface is provided on a vital sign monitoring interface, but also a blood pressure parameter value and a position information can be stored correspondingly.

For ease of understanding, this disclosure provides the following schematic diagrams of several vital sign monitoring interfaces, and the specific implementation process of the physiological parameter measurement method is described in combination with the schematic diagrams.

In an embodiment, a vital sign monitoring interface includes at least two measurement start control and at least two measurement position information icon which is used to indicate a posture information that needs to be maintained during blood pressure measurement. There is a pre-set correspondence relationship between the position information icon and different measurement start controls.

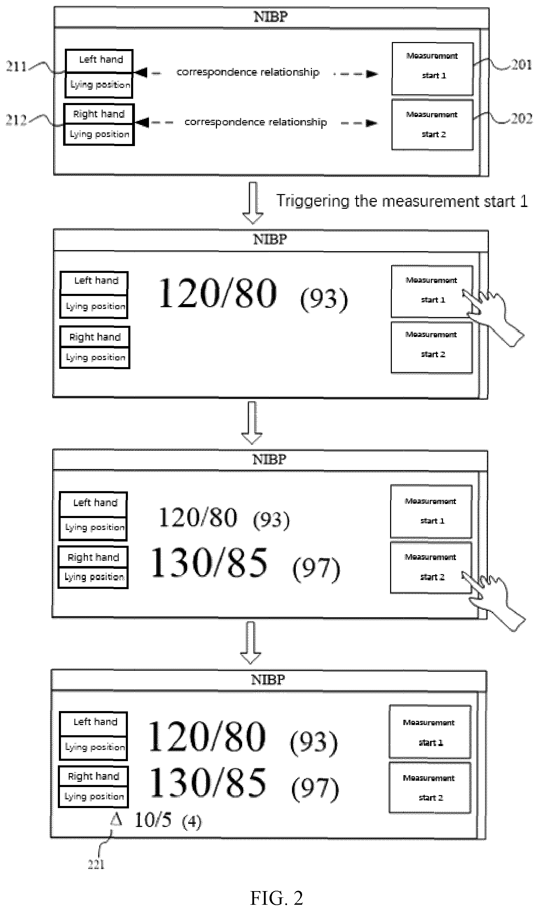

As Figure 2, the vital sign monitoring interface includes two measurement start controls ang two position information icons which indicate left-hand and right-hand recumbent positions. The measurement start control 201 corresponds to the position information icon 211 and the measurement start control 202 corresponds to the position information icon 212. The vital sign monitoring interface display blood pressure measurement methods, as shown in Figure 2, NIBP (automated non-invasive blood pressure) is displayed in each interface.

When measuring blood pressure measurement of a monitored object by a vital sign monitoring, the medical staff instructs the monitored object to be in a lying position according to the measurement requirements, wears a blood pressure measurement accessory to the left hand of the monitored object, and then triggers a measurement start control 201 to make the blood pressure measurement accessory collect blood pressure parameter values.

When the vital signs monitoring device detects that the measurement start control 201 is triggered, the position information icon 211 corresponding to the measurement start control 201 indicate left-hand lying position that is determined by the above correspondence relationship, and the collected blood pressure parameter value is displayed in an area that associated with the left-hand lying position, as shown in FIG.2, the blood pressure parameter value is displayed in the same row as the left-hand lying position information icon. When it is considered that the blood pressure parameter value is incorrectly and needs to be re-measured, a user can trigger corresponding measurement start control again to perform measurement and display again.

In the same way, the measurement start control 202 can be triggered to complete the measurement of the blood pressure parameter value in the right-hand lying position. Further, when there are many the target blood pressure parameter value and each target blood pressure parameter value corresponding to different target position information, displaying the target position information corresponding to the target pressure parameter value that collected at first time and the last time. Specifically, when the user triggers the measurement start control, assuming that the blood pressure parameter value corresponds to the previous measurement start control has been measured and displayed, and then adjust the display style of the blood pressure parameter value corresponding to the previous measurement start control to make it different from the display style of the blood pressure measurement parameter to be displayed corresponding to the currently triggered measurement start control for prompting the user which position corresponding to the currently blood pressure parameter value is. As FIG.2 shown, when the user triggers the measurement start control 202, reduce the font size of the blood pressure parameter value corresponding to the measurement start control 201. Of course, in actual applications, other types of adjustments can also be made to the display style, which is not specifically limited in this disclosure. As FIG.2 shown, after measuring the blood pressure value in two posture by triggering two measurement start control, two blood pressure parameter value can be displayed in the vital sign monitoring interface at same time.

It should be noted that the number of the measurement start control and the position information icon is not limited to two, but can also be other values set by the user according to clinical application requirements (for example, the number of measurements that need to be performed during vital point testing), which is not specifically limited in this disclosure. And the correspondence relationship between the measurement start control and the position information icon is not limited to one-to-one correspondence, but can also be a one-to-many relationship. For example, the measurement start control 201 corresponds to the left-hand lying position information icon and right-hand lying position information icon, and the measurement start control 201 corresponds to the left-hand standing position information icon and the right-hand standing position information icon. In this case, the vital sign monitoring equipment needs to be equipped with multiple blood pressure measurement devices, so that after the user triggers the measurement start control, the blood pressure parameter values in multiple positions can be measured at the same time.

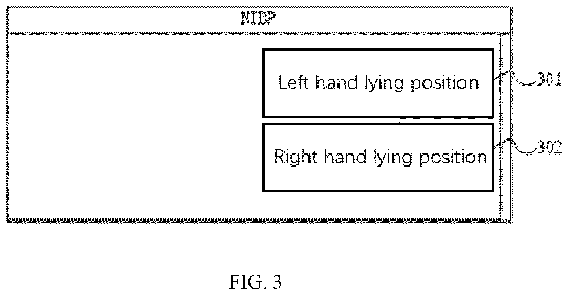

In the embodiment, the vital sign monitoring interface may not include the position information icon, but the position information is displayed by the measurement start control. For example, as FIG.3 shown that the text information displayed by the measurement start control 301 is "left hand lying position", and the text information displayed by the measurement start control 302 is "right hand lying position", so that after the user triggers the measurement start control, the target position information corresponding to the triggered measurement start control is determined according to the correspondence relationship between the measurement start control and the position information.

In the embodiment, the measurement start control is not limited to the above form, that is, multiple measurement start control types can be the same or different. Specifically, the measurement start control can include commonly used measurement start control and measurement start control increase. The former measurement start control is used to measure the blood pressure parameter value in a certain conventional position, and the latter measurement start control is used to measure the blood pressure parameter value in an unconventional position. Of course, the conventional position and the unconventional position can be pre-set according to actual requirements. Further, the vital sign monitoring interface may also include common position information icons, common measurement start controls are associated with common position information icons, and common position information icons are used to indicate pre-set conventional position information that needs to be maintained during blood pressure measurement. The vital sign monitoring interface can be used to the situation that need multiple measurement requirement. For example, the vital sign monitoring equipment is usually used during daily rounds when the medical staff often only need to measure the blood pressure parameters of the monitored object in a certain position. But when using the vital sign monitoring equipment to evaluate the admitted patient, it is necessary to measure the blood pressure parameter values of the patient in a variety of positions to comprehensively evaluate the patient's blood pressure status. In this case, medical staff can add blood pressure measurement procedures in other positions through the vital signs monitoring interface. In response to the triggering operation input, displaying the position information setting interface; receiving the position information that set for the target measurement start control in position information setting interface and determining that the position information is the target position information corresponding to the target measurement start control.

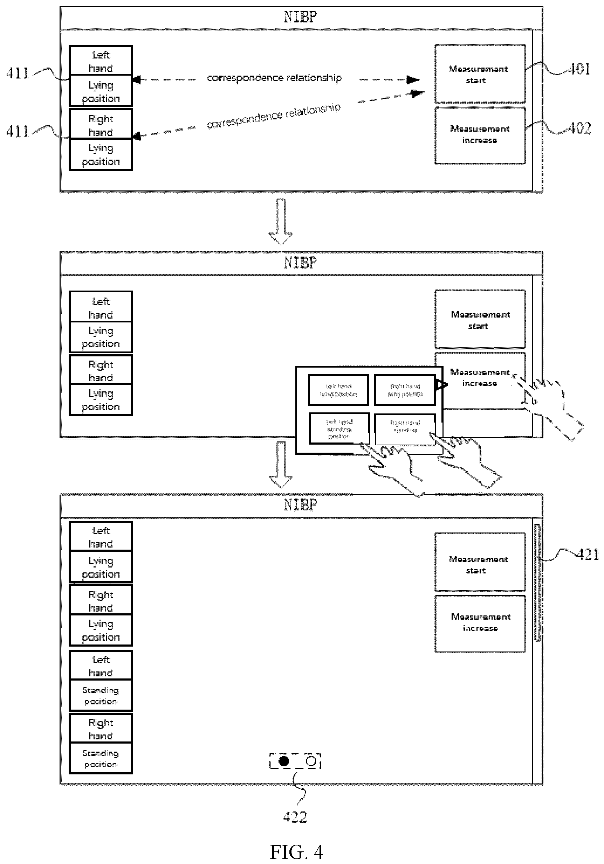

As FIG.4 shown that the vital sign monitoring interface includes a common measurement start control 401, two common position information icons 411, and an additional measurement start control 402. The common measurement start control 401 is associated with the common position information icon 411, and the common position information icon 411 indicates the positions of the left-hand lying position and right-hand lying positions that need to be maintained during blood pressure measurement. Of course, the number of the common measurement start control and common position information icon corresponding to the common measurement start control is not limited to the above number, and may also be other numbers.

The medical staff can trigger the common measurement start control to collect the blood pressure parameter values in the left-hand lying position and the blood pressure parameter values in the right-hand lying position of the monitored object. When the medical staff wants to add a measurement in another position, they can trigger a new measurement start control. As FIG.4 shown that when detecting the triggering operation by the user through the added measurement start control, a setting interface containing candidate position information pops up, and the user can select a certain position information in the setting interface as the new position information. In the embodiment of the disclosure, the added position information is determined as the target position information corresponding to the added measurement start control. After the blood pressure measurement increase collects the target blood pressure parameter value, the target blood pressure parameter value can be associated with the target position information. It should be noted that the user can add multiple position information at a time, or add one position information at a time. In addition, this disclosure can add position information in other ways than the display interface, for example, through physical buttons. And the process of adding position information can be performed before the blood pressure parameter value is collected, or can be performed at the same time or after it.

In this case, when displaying the target blood pressure parameter value, a target position information icon used to indecate the target position information can be generated; displaying the target position information icon, determining the displaying area corresponding to the target position information in the vital sign monitoring interface and displaying the target blood parameter value in the displaying area corresponding to the target position information, for example displaying the blood pressure parameter value in an area corresponding to the display area of the target position. As FIG.4 shown that assuming that the position information selected by the user is the left-hand of the position and the right-hand of the position, the position information icons corresponding to the two position information are added in the vital sign monitoring interface. The collected target blood pressure parameter value can be displayed in the corresponding position of the associated position information icon. When the display area of the vital sign monitoring interface is small, the added position information icon can be displayed on another screen. A draggable sidebar 421 is generated on the right side of the interface or a screen prompt icon 422 is generated at the bottom of the interface. The user can drag the sidebar 421 or slide the screen left and right to view the display data of another screen.

It should be noted that the embodiments of the disclosure are not limited to expressing position information in words, but can also be expressed in a graphical manner. For example, an image of a person lying flat may be used to indicate a lying position, and an image of a standing person may be used to indicate a standing position. In addition, for each embodiment that includes both the position information icon and the measurement start control, the measurement start control can be combined with the position information icon. Specifically, as FIG.3 shown that the position information icon can be displayed by the measurement start control. Or the measurement start control may not only the virtual buttons in the interface, but also be physical buttons such as buttons set on the equipment body or buttons set on a blood pressure measurement accessory. That is to say, the user can initiate blood pressure measurement by triggering a physical button. Furthermore, the blood pressure measurement accessory can also collect the pulse value while collecting the blood pressure value and the pulse value and the blood pressure parameter value can be displayed together. As FIG.2 and FIG.4 shown that indicating the pulse value that measured same time by using parentheses in the vital sign monitoring interface.

In order to further improve the user's flexibility in the operation of the vital sign monitoring equipment, the user can also modify the content of the target position information. Specifically, the user can input an instruction that modify the target position information, for example, click the target position information icon to modify, and in response to the modification instruction that modify the target position information, and then obtain the modified position information. And use the modified position information to replace the target position information

In addition to displaying the blood pressure measurement values in different positions, the vital signs monitoring interface can also calculate and display the difference of the blood pressure measurement values. Specifically, in an embodiment, after establishing multiple correspondence relationship between the blood pressure measurement value and the target position information, determining the target position information associate with pre-set the blood pressure difference value calculation rule; and displaying the blood pressure difference value in the vital sign monitoring interface.

Different blood pressure difference value calculation rules require different positions for calculating the blood pressure difference value. For example, the blood pressure difference value between the left-hand lying position and the right-hand lying position can be calculated, or the blood pressure difference value between the left-hand lying position and the left-hand standing position can be calculated. Therefore, the user can pre-set the blood pressure difference value calculation rule in the setting interface, so that in actual applications, the position information that needs to be involved in the calculation can be determined according to the blood pressure difference value calculation rule, calculating the difference between the blood pressure value corresponding to the position information that needs to be involved in the calculation. Further, the blood pressure difference value can be displayed on the vital sign monitoring interface. As FIG. 2 shown that calculating the difference between the blood pressure measurement value of the left-hand lying position and the right-hand lying position, and displaying the blood pressure difference value between the blood pressure at the position corresponding to the triangular icon 221. Among them, in addition to using the triangle icon, other graphics or text can also be used to indicate the difference value.

Further, when the blood pressure difference value satisfies the blood pressure alarm condition, the blood pressure difference value is displayed according to a pre-set alarm pattern. For example, the blood pressure difference value may be marked red or a red background to remind the user that the blood pressure difference value is abnormal.

In actual applications, there may be situations where the position of the monitored object when collecting blood pressure parameter values is inconsistent with the position information corresponding to the triggered measurement start control. For example, the user wears the blood pressure measurement accessory on the right hand of the monitored object in the lying position, but the position information corresponding to the started measurement start control is the left-hand lying position. In this case, the blood pressure data recorded will be wrong. In order to avoid such operation mistakes as much as possible, the embodiments of the disclosure may monitor the position and give alarm prompts when detects error operations. Specifically, in one embodiment, before establishing the correspondence relationship between the target blood pressure parameter value and the target position information, determining the actual position information of the monitored object when the target blood pressure parameter value is collected and determining whether the actual position information is consistent with the target position information. Of course, the actual position information of the monitored object can be determined in various ways. For example, the blood pressure measurement accessory is used to a certain measurement position, and the internal identification data of the blood pressure measurement accessory can be obtained, and the measurement position recorded in the internal identification data is used as the actual position information of the monitored object during the measurement of the blood pressure measurement accessory; another example is to obtain the image data of the monitored object, and identify the actual position information of the monitored object through image recognition technology; another example is that the position information of the monitored object detected by other equipment such as smart beds can be used as the actual position information, etc. When the actual position information is consistent with the target position information, establishing a correspondence relationship between the target blood pressure parameter value and the target position information. When the actual position information is inconsistent with the target position information, outputting the prompt information.

It should be noted that before the blood pressure measurement, the measurement mode can also be selected that include a variety of options: select the inflation measurement mode or the deflation measurement mode according to the size or type of the cuff; automatically select measurement modes according to patient information such as age, for example, configure different measurement modes for adults, newborns, and children; configure different patient modes according to different patient types, such as adopting inflation measurement mode for hypertensive patients to avoid discomfort caused by deflation measurement mode; Set different measurement modes in different countries, user can choose according to the area user is in.

It should be noted that when the vital sign monitoring equipment is equipped with multiple different blood pressure measurement accessory (blood pressure measurement module), different measurement start control corresponds to different blood pressure measurement accessory, and the vital sign monitoring interface may include prompt information used to indicate the correspondence relationship between the measurement start control and the blood pressure measurement accessory, such as text prompt information or graphical prompt information.

In the actual implementation process, the method for measuring physiological parameter may need to be executed multiple times under different conditions, and different application scenarios may require to adjust or customize some configuration information in the method for measuring physiological parameter. In order to improve the measurement efficiency, user can generate a measurement mode for a certain defined method for measuring physiological parameter and store it. When the user needs to use the measurement mode for measurement, the user can directly call the measurement mode, skipping the process that the user adjusts or customize the configuration information. Specifically, a physiological parameter measurement mode is generated and stored, and the physiological parameter measurement mode includes at least one of the following: the vital sign monitoring interface, the correspondence relationship between the target measurement start control and the target position information, and the correspondence relationship between the blood pressure measurement accessory and the target position information; in response to the call instruction to the physiological parameter measurement mode, perform the physiological parameter measurement model.

Among them, the vital sign monitoring interface includes the target measurement start control and storing the correspondence relationship between the target measurement start control and the target position information can skip that step that define the correspondence relationship. After the user selects the target measurement start control, the position information corresponding to the target measurement start control can be clarified according to the correspondence relationship.

Storing the correspondence relationship between the blood pressure measurement accessory and the target position information, after obtaining the blood parameter value by the blood pressure measurement accessory, determining that the target blood pressure value measured in which position information by the correspondence relationship and the blood pressure parameter value is directly displayed at the position corresponding to the target position information.

It should be noted that the call flag corresponding to the physiological parameter measurement mode can be set, and the physiological parameter measurement mode can be found through the call flag. For example, the call flag may include the identification of the monitored object, such as name, hospitalization code, age, type, etc., so that different monitored object can store different physiological parameter measurement mode. When the monitored object is measured, call the physiological parameter measurement mode corresponding to identification of the monitored object. Or, the call flag can also include the identification of the medical staff such as name, job number, etc., so that different medical staff can store the physiological parameter measurement mode corresponding to their own habits. When the medical staff needs to perform the measurement work, call the physiological parameter measurement mode corresponding to the identification of the medical. Alternatively, the call flag may also include monitoring scene identifications such as daily measurement scenes, hospital admission measurement scenes, etc., and call the physiological parameter measurement mode according to the actual use scene. Or based on the above revelation, the call flag may also be other call identifiers that can be conceived by those skilled in the art that can distinguish different physiological parameter measurement modes.

Each of the above embodiments is described by taking the blood pressure as a physiological parameter as an example. However, other physiological parameters may also need to be measured in different positions. Therefore, measuring for the blood pressure parameter value can be further extended to other physiological parameter with different measurement requirements, such as body temperature, pulse, and so on. The specific embodiment is: displaying the vital sign monitoring interface, the vital signs monitoring interface includes at least two measurement start controls.

In response to the triggering operation input by the user through the target measurement start control of at least two measurement start controls, determining the target position information corresponding to the target measurement start control, and the target position information is used to indicate the target posture and/or the target measurement position of the monitored object that needs to be maintained during measuring the physiological parameter of the monitored object.

Obtain the target physiological parameter value of the monitored object collected by the physiological parameter measurement accessory.

Establish the correspondence relationship between the target physiological parameter value and the target position information.

Display the target physiological parameter value by indicating the correspondence relationship between the target physiological parameter value and the target position information.

It is understandable that when measure a certain physiological parameter, use the physiological parameter measurement accessory corresponding to the physiological parameter, such as using a body temperature probe to collect body temperature values, and using a pulse measuring accessory to collect pulse values. Determining the target position information for body temperature measurement can be achieved in the following ways: for example, the body temperature measurement part can be automatically determined according to the type of body temperature probe connected to the equipment, such as oral cavity, forehead temperature, armpit, anal body temperature, etc.

Another example, determining by the way that the image recognition module identify the appearance outline or image of the body temperature probe.

Another example, determining by the identification structure set on the body temperature probe, such as identification code, connection interface, etc.

Another example, determining by decoding the information sent by the body temperature probe. Of course, these methods for determining the target position information can also be applied to other measurement accessory, such as blood pressure measurement accessory, to determine the target position information of the other physiological parameter.

Furthermore, measuring the physiological parameter many times may not be related to the position information, but are parameter data collected at multiple different measurement moments, and the multiple parameter data can be displayed on the vital sign monitoring interface. Specifically, the vital sign monitoring interface is displayed, the vital sign monitoring interface includes a measurement start control, and the measurement start control corresponds to the target physiological parameter.

In respond to the triggering operation input by the user through the measurement start control, control the measurement physiological parameter accessory to collect the parameter data of the target physiological parameter of the monitored object multiple times at different measurement moments.

In the vital sign monitoring interface, display the parameter data of the target physiological parameters of the monitored object collected multiple times.

In the embodiment of the disclosure, after the user triggers the measurement start control, the vital sign monitoring equipment can automatically collect multiple collections of the monitored object at different times, for example, the blood pressure value is collected every five minutes, and the blood pressure parameter values collected three times are collected in total. The multiple parameter data can be displayed in the vital sign monitoring interface for the user to view.

In order to ensure the application and realization of the above method in practice, this disclosure provides the following vital sign monitoring equipment. The vital sign monitoring equipment may be a medical equipment with a monitoring function, such as a monitor, etc.

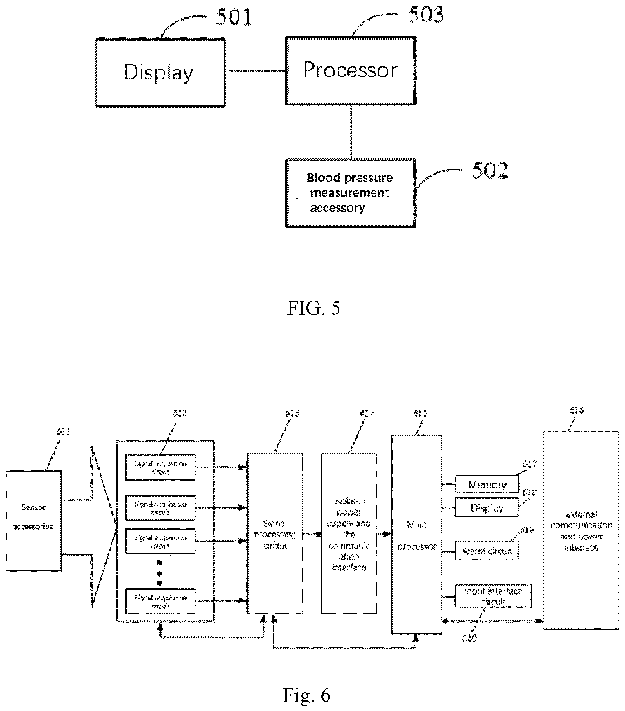

As FIG.5 shown that a vital sign monitoring equipment, including: a display 501, a blood pressure measurement accessory 502, and a processor 503.

A display 501 that is configured to display a vital sign monitoring interface, and the vital sign monitoring interface includes at least two measurement start control; in the vital sign monitoring interface, a target blood pressure parameter value is displayed by indicating a correspondence relationship between the target blood pressure parameter value and a target position information.

A blood pressure measurement accessory 502 that is used to collect the target blood pressure parameter value of a monitored object.

A processor 503 that configured to: determine the target position information corresponding to the target measurement start control in response to a triggering operation input by a user through a target measurement start control of the at least two measurement start control; wherein the target position information is used to indicate a target posture that a monitored object needs to maintain and/or a target measurement position of a monitored object when performing blood pressure measurement.

Obtain the target blood pressure parameter value of the monitored object collected by the blood pressure measurement accessory.

Establish a correspondence relationship between the target blood pressure parameter value and the target position information.

In the embodiment, the vital sign monitoring equipment further including: a memory, which is used to store the target blood pressure parameter value, the target position information, and the correspondence relationship between the target blood pressure parameter value and the target position information.

In the embodiment, the processor 503 display the target blood pressure parameter value by indicating the correspondence relationship between the target blood pressure parameter value and the target position information. It is specifically used to determine a display area, which corresponds to the target position information, in the vital sign monitoring interface, and display the target blood pressure parameter value in the display area corresponding to the target position information.

In the embodiment, the vital sign monitoring interface includes at least two posture information icons, which are used to indicate posture information that needs to be maintained during blood pressure measurement, and there are pre-sets correspondence relationship between different posture information icons and different measurement start control.

In the embodiment, the processor 503 is further configured to obtain the pre-set correspondence relationship between a measurement start control and position information.

Determining, according to the pre-set correspondence relationship, the target position information corresponding to the target measurement start control.

In the embodiment, the measurement start control includes common measurement start control, common position information icon and measurement start increase control; wherein the common measurement start control is associated with the common position information icon, and the common position information icon is used to indicate a pre-set conventional position information that needs to be kept during blood pressure measurement.

In an embodiment, the processor 503 determine the target position information corresponding to the target measurement start control, it is specifically configured to: when the target measurement start control is the measurement start increase control, display a position information setting interface.

Receive the position information set by the user for the target measurement start control based on the position information setting interface.

Determine the set position information as the target position information corresponding to the measurement start increase control.

In the embodiment, the processor 503 display the target blood pressure parameter value by indicating the correspondence relationship between the target blood pressure parameter value and the target position information, it is specifically configured to: when the target measurement start control corresponding to the target position information is the measurement start increase control, generate a target position information icon for representing the target position information.

Display the target position information icon, determine a display area corresponding to the target position information in the vital sign monitoring interface, and displaying the target blood pressure parameter value in the display area corresponding to the target position information.

In the embodiment, the processor 503 is further configured to determine the target position information associated with a pre-set blood pressure difference calculation rule after establishing the correspondence relationship between the target blood pressure parameter values and the target position information; and calculate a blood pressure difference between the target blood pressure parameter values corresponding to the associated target position information, according to the blood pressure difference calculation rule.

The display 501 is further configured to display the blood pressure difference in the vital sign monitoring interface.

In the embodiment, the display 501 is configured to display the blood pressure difference according to a pre-set alarm pattern when the blood pressure difference meets a blood pressure alarm condition.

In the embodiment, the processor 503 is configured to: display a last collected target blood pressure parameter value and the target position information corresponding to the last collected target blood pressure parameter value in different display styles when there are multiple target blood pressure parameter values and each target blood pressure parameter value corresponds to different target position information.

In the embodiment, the processor 503 is configured to obtain modified position information in response to a modification instruction to modify the target position information and then replace the target position information with the modified position information.

In the embodiment, the processor 503 is further configured to determine an actual position information of the monitored object when collecting the target blood pressure parameter value, and determine whether the actual position information is consistent with the target position information before establishing the correspondence relationship between the target blood pressure parameter value and the target position information.

The processor 503 is configured to establish the correspondence relationship between the target blood pressure parameter value and the target position information when the actual position information is consistent with the target position information.

In the embodiment, the processor 503 is further configured to output prompt information, when the actual position information is inconsistent with the target position information.

In the embodiment, each measurement start control corresponds to a different blood pressure measurement accessory, and the vital sign monitoring interface includes prompt information for indicating the correspondence relationship between the measurement start control and the blood pressure measurement accessory.

In the embodiment, the processor 503 is further configured to receive the triggering operation input by the user through the target measurement start control of the at least two measurement start control before determining the target position information corresponding to the target measurement start control in response to the triggering operation input by the user through the target measurement start control in the at least two measurement star control; the trigger operation is generated by tapping a physical button for blood pressure measurement by the user.

In the embodiment, the processor 503 is further configured to generate and store a physiological parameter measurement mode, and execute the physiological parameter measurement mode in response to an instruction to invoke the physiological parameter measurement mode; the physiological parameter measurement mode includes at least one of: the vital sign monitoring interface, the correspondence relationship between the target measurement start control and the target position information, and the correspondence relationship between the blood pressure measurement accessory and the target position information.

In addition, an embodiment of the disclosure also provides a vital sign monitoring equipment, including: a display configured to display a vital sign monitoring interface, the vital sign monitoring interface including at least two measurement start control; in the vital sign monitoring interface, a target physiological parameter value is displayed by representing a correspondence relationship between the target sign parameter value and a target position information.

A physiological parameter measurement accessory used to collect the target physiological parameter value of a monitored object.

A processor that is configured to: determine the target position information corresponding to the target measurement start control in response to a triggering operation input by a user through a target measurement start control of the at least two measurement start control; wherein the target position information is used to indicate a target posture that the monitored object needs to maintain and/or a target measurement position of the monitored object when performing physiological parameter measurement.

Obtain the target physiological parameter value of the monitored object collected by the physiological parameter measurement accessory.

Establish a correspondence relationship between the target physiological parameter value and the target position information.

In addition, an embodiment of the disclosure also provides a vital sign monitoring equipment, including: a display configured to display a vital sign monitoring interface, wherein the vital sign monitoring interface includes a measurement start control that corresponds to a target physiological parameter; the parameter data of the target physiological parameter collected multiple times are displayed in the vital sign monitoring interface.

A processor configured to, in response to a triggering operation input by a user through the measurement start control, control a physiological parameter measurement accessory to collect the parameter data of the target physiological parameter of the monitored object multiple times at different measurement moments.

In addition, an embodiment of the disclosure also provides a readable storage medium on which a computer program is stored, and when the computer program is loaded and executed by a processor, the physiological parameter measurement method provided in any of the foregoing embodiments is implemented. An electronic device such as a mobile terminal can be provided with the readable storage medium, and the computer program can be loaded and executed by the processor to implement the physiological parameter measurement method provided in any of the foregoing embodiments.

A specific implementation product of vital sign monitoring equipment can be a monitor. A specific example of a monitor is shown in FIG.6. FIG.6 provides a system framework diagram of a parameter processing module in a multi-parameter monitor.

The multi-parameter monitor has an independent housing. The housing panel has a sensor interface area, which integrates multiple sensor interfaces for connecting with external physiological parameter sensor accessories 611. The housing panel also includes a small IXD display area and display 618, input interface circuit 620 and alarm circuit 619 (such as LED alarm area) and so on. The parameter processing module is used for external communication and power supply interface for communicating with the host and taking power from the host. The parameter processing module also supports an external plug-in parameter module. The plug-in monitor host can be formed by inserting the parameter module as a part of the monitor, or it can be connected to the host via a cable. The external plug-in parameter module is used as an external accessory of the monitor. In addition, the multi-parameter monitor includes a memory 617 for storing computer programs and various data generated during the related monitoring process.

The internal circuit of the parameter processing module is placed in the housing, as shown in FIG.6,including at least two physiological parameter corresponds to signal acquisition circuit 612, front-end signal processing circuit 613 and main processor 615.

The main processor 615 can implement the processing-related steps in each of the above-mentioned methods for monitoring apnea events.

The signal acquisition circuit 612 can be selected from an ECG circuit, a breathing circuit, a body temperature circuit, a blood oxygen circuit, a non-invasive blood pressure circuit, an invasive blood pressure circuit, etc., the signal acquisition circuits 612 are respectively electrically connected to the corresponding sensor interface for electrical connection to a sensor accessory 611 corresponding to different physiological parameters that output end is coupled to the front-end signal processor, the communication port of the front-end signal processor is coupled to the main processor, and the main processor is electrically connected to the external communication and power supply interface.

Various physiological parameter measurement circuits can use common circuits in the prior art. The front-end signal processor completes the sampling and analog-to-digital conversion of the output signal of the signal acquisition circuit, and outputs control signals to control the measurement process of physiological signals. These parameters include but not limited to: ECG, respiration, body temperature, blood oxygen, non-invasive blood pressure and invasive blood pressure parameters.

The front-end signal processor can be realized by a single-chip microcomputer or other semiconductor devices, and can also be realized by an ASIC or FPGA. The front-end signal processor can be powered by an isolated power supply, and the sampled data is simply processed and packaged, and then sent to the main processor through the isolated communication interface. For example, the front-end signal processor circuit can be coupled to the main processor 615 through the isolated power supply and the communication interface 614.

The reason that the front-end signal processor is powered by an isolated power supply is that the DC/DC power supply isolated by a transformer plays a role in isolating the patient from the power supply equipment. The main purpose is: 1. the patient leakage current is small enough; 2. prevent the voltage or energy during defibrillation or electrosurgical application from affecting the boards and devices of the intermediate circuit such as the main control board (guaranteed by creepage distance and electrical clearance).

The main processor completes the calculation of physiological signs parameters, and sends the calculation results and waveforms of the parameters to the host (such as the host with a display, PC, central station, etc.) through the external communication and power interface.

The external communication and power interface 616 can be selected of that one or a combination of LAN interfaces composed of Ethernet, Token Ring, Token Bus, and the backbone fiber distributed data interface (FDDI) of these three networks. and It also can be one or a combination of wireless interfaces such as infrared, Bluetooth, wifi, and WMTS communication, or one or a combination of wired data connection interfaces such as RS232 and USB.

The external communication and power interface 616 may also be one or a combination of a wireless data transmission interface and a wired data transmission interface. The host can be any computer equipment such as the host of the monitor, the electrocardiograph, the ultrasonic diagnostic apparatus, the computer, etc., and the matching software can be installed to form a monitoring device. The host can also be a communication device, such as a mobile phone, and the parameter processing module sends data to a mobile phone that supports Bluetooth communication through a Bluetooth interface to realize remote data transmission.

This document is described with reference to various exemplary embodiments. However, those skilled in the art will recognize that changes and modifications can be made to the exemplary embodiments without departing from the scope of this document. For example, various operation steps and components used to perform the operation steps can be implemented in different ways according to specific applications or considering any number of cost functions associated with the operation of the system (for example, one or more steps can be deleted, modify or incorporate into other steps).

The terms "first", "second", etc. in the specification and claims herein and the above-mentioned drawings are used to distinguish different objects, rather than to describe a specific order. In addition, the terms "including" and "having" and any variations thereof are intended to cover non-exclusive inclusions. For example, a process, method, system, product, or equipment that includes a series of steps or units is not limited to the listed steps or units, but optionally includes unlisted steps or units, or optionally also includes other steps or units inherent to these processes, methods, or equipment.

In addition, as understood by those skilled in the art, the principles herein can be reflected in a computer program product on a computer-readable storage medium, which is pre-installed with computer-readable program code. Any tangible, non-transitory computer-readable storage medium can be used, including magnetic storage devices (hard disks, floppy disks, etc.), optical storage devices (CD-ROM, DVD, Blu Ray disks, etc.), flash memory and/or the like. These computer program instructions can be loaded on a general-purpose computer, a specialpurpose computer, or other programmable data processing equipment to form a machine, so that these instructions executed on the computer or other programmable data processing device can generate a device that realizes the specified function.

These computer program instructions can also be stored in a computer-readable memory, which can instruct a computer or other programmable data processing equipment to run in a specific manner, so that the instructions stored in the computer-readable memory can form a piece of manufactured products, including realizing devices that realize designated functions. Computer program instructions can also be loaded on a computer or other programmable data processing equipment, thereby executing a series of operation steps on the computer or other programmable equipment to produce a computer-implemented process, so that instructions of the execution of the computer or other programmable equipment can provide steps for implementing specified functions.

The foregoing detailed description has been described with reference to various embodiments. However, those skilled in the art will recognize that various modifications and changes can be made without departing from the scope of this disclosure. Therefore, the consideration of this disclosure will be in an illustrative rather than restrictive sense, and all these modifications will be included in its scope. Likewise, the advantages, other advantages, and solutions to problems of the various embodiments have been described above. However, benefits, advantages, solutions to problems, and any elements that can produce these, or make them more specific, should not be construed as critical, necessary, or necessary. The term "including" and any other variants thereof used in this article are non-exclusive inclusions. Such a process, method, article or device that includes a list of elements not only includes these elements, but also includes those that are not explicitly listed or are not part of the process, methods, systems, articles or other elements of equipment. In addition, the term "coupled" and any other variations thereof used herein refer to physical connection, electrical connection, magnetic connection, optical connection, communication connection, functional connection and/or any other connection.

The above examples only express several implementation modes, and the descriptions are more specific and detailed, but they should not be understood as limiting the scope of the patent of the present invention. It should be pointed out that for those of ordinary skill in the art, without departing from the concept of the present invention, several modifications and improvements can be made, and these all fall within the protection scope of the present invention. Therefore, the protection scope of the patent of the present invention should be subject to the appended claims.

## Claims

1. An equipment for monitoring vital sign, comprising:
a display that is configured to display a vital sign monitoring interface, and the vital sign monitoring interface comprises at least two measurement start controls; in the vital sign monitoring interface, a target blood pressure parameter value is displayed by indicating a correspondence relationship between the target blood pressure parameter value and a target position information;
a blood pressure measurement accessory that is used to collect the target blood pressure parameter value of a monitored object;
a processor that is configured to:
determine the target position information corresponding to the target measurement start controls in response to a user's triggering operation input by a user through a target measurement start control of the at least two measurement start control; wherein the target position information is used to indicate a target posture and/or a monitored object's target measurement position of a monitored object when performing blood pressure measurement;
obtain the target blood pressure parameter value of the monitored object collected by blood pressure measurement accessory; and
establish a correspondence relationship between the target blood pressure parameter value and target position information;

2. The equipment of claim 1, further comprising:
a memory, which is used to store the target blood pressure parameter value, the target position information, and the correspondence relationship between the target blood pressure parameter value and the target position information.

3. The equipment of claim 1, wherein:
the processor is further configured to:
determine a display area, which corresponds to the target position information, in the vital sign monitoring interface, and display the target blood pressure parameter value in the display area corresponding to the target position information.

4. The equipment of claim 1, wherein:
the vital signs monitoring interface comprises at least two position information icons, which are used to indicate position information that needs to be maintained during blood pressure measurement, and there are pre-sets correspondence relationship between different position information icons and different measurement start control; and
the processor is further configured to:
obtain the pre-set correspondence relationship between a measurement start control and position information;
determine, according to the pre-set correspondence relationship, the target position information corresponding to the target measurement start control.

5. The equipment of claim 1, wherein:
the measurement start control comprises common measurement start control, common position information icon and measurement start increase control; wherein the common measurement start control is associated with the common position information icon, and the common position information icon is used to indicate a pre-set conventional position information that needs to be kept at the time during measuring blood pressure measurement.

6. The equipment of claim 5, wherein the processor is further configured to:
when the target measurement start control is the measurement start increase control, display a position information setting interface;
receive the position information set by the user for the target measurement start control based on the position information setting interface;
determine the set position information as the target position information corresponding to the measurement start increase control.

7. The equipment of claim 6, wherein the processor is further configured to:
when the target measurement start control corresponding to the target position information is the measurement start increase control, generate a target position information icon for representing the target position information;
display the target position information icon, determine a display area corresponding to the target position information in the vital signs monitoring interface, and displaying the target blood pressure parameter value in the display area corresponding to the target position information.

8. The equipment of claim 1, wherein the processor is further configured to:
determine the target position information associated with a pre-set blood pressure difference calculation rule after establishing the correspondence relationship between the target blood pressure parameter values and the target position information; and calculate a blood pressure difference between the target blood pressure parameter values corresponding to the associated target position information, according to the blood pressure difference calculation rule;
the display is further configured to display the blood pressure difference in the vital sign monitoring interface.

9. The equipment of claim 1, wherein the processor is further configured to:
display a last collected target blood pressure parameter value and the target position information corresponding to the last collected target blood pressure parameter value in different display styles when there are multiple target blood pressure parameter values and each target blood pressure parameter value corresponds to different target position information.

10. The equipment of claim 1, wherein:
the processor is further configured to determine an actual position information of the monitored object when collecting the target blood pressure parameter value, and determine whether the actual position information is consistent with the target position information before establishing the correspondence relationship between the target blood pressure parameter value and the target position information;
the processor is configured to establish the correspondence relationship between the target blood pressure parameter value and the target position information when the actual position information is consistent with the target position information.

11. The equipment of claim 1, wherein:
each measurement start controls corresponds to a different blood pressure measurement accessory, and the vital sign monitoring interface comprises prompt information for indicating the correspondence relationship between the measurement start control and the blood pressure measurement accessory.

12. The equipment of claim 1, wherein:
the processor is further configured to receive the triggering operation input by the user through the target measurement start control of the at least two measurement start control before determining the target position information corresponding to the target measurement start control in response to the triggering operation input by the user through the target measurement start control in the at least two measurement star control; the trigger operation is generated by tapping a physical button for blood pressure measurement by the user.

13. The equipment of claim 1, wherein:
the processor is further configured to generate and store a physiological parameter measurement mode, and execute the physiological parameter measurement mode in response to an instruction to invoke the physiological parameter measurement mode; the physiological parameter measurement mode comprises at least one of: the vital sign monitoring interface, the correspondence relationship between the target measurement start control and the target position information, and the correspondence relationship between the blood pressure measurement accessory and the target position information.

14. An equipment for monitoring vital sign, comprising:
a display configured to display a vital sign monitoring interface, the vital sign monitoring interface comprising at least two measurement start controls; in the vital sign monitoring interface, a target physiological parameter value is displayed by representing a correspondence relationship between the target sign parameter value and the target position information;
a physiological parameter measurement accessory used to collect the target physiological parameter value of a monitored object;
a processor that is configured to:
determine the target position information corresponding to the target measurement start control in response to a triggering operation input by a user through a target measurement start control of the at least two measurement start control; wherein the target position information is used to indicate a target posture that the monitored object needs to maintain and/or a target measurement position of the monitored object when performing physiological parameter measurement;
obtain the target physiological parameter value of the monitored object collected by the physiological parameter measurement accessory; and
establish a correspondence relationship between the target physiological parameter value and the target position information.

15. An equipment for monitoring vital sign, comprising:
a display configured to display a vital sign monitoring interface, wherein the vital sign monitoring interface comprises a measurement start control that corresponds to a target sign parameter; the parameter data of the target physiological parameter collected multiple times are displayed in the vital sign monitoring interface;
a processor configured to, in response to a triggering operation input by a user through the measurement start control, control a physiological parameter measurement accessory to collect the parameter data of the target physiological parameter of the monitored object multiple times at different measurement moments.
